(19) Europäisches Patentamt
European Patent Office
Office européen des brevets

(11) Veröffentlichungsnummer: **0 304 016**
**A1**

# (12) EUROPÄISCHE PATENTANMELDUNG

(21) Anmeldenummer: **88113293.0**

(22) Anmeldetag: **17.08.88**

(51) Int. Cl.4: **C07C 125/06**

(30) Priorität: **19.08.87 DE 3727665**

(43) Veröffentlichungstag der Anmeldung:
**22.02.89 Patentblatt 89/08**

(84) Benannte Vertragsstaaten:
**CH DE FR GB LI NL**

(71) Anmelder: **CASSELLA Aktiengesellschaft**
**Hanauer Landstrasse 526**
**D-6000 Frankfurt am Main 61(DE)**

(72) Erfinder: **Engelhardt, Friedrich, Dr.**
**Hünfelder Strasse 20**
**D-6000 Frankfurt am Main(DE)**
Erfinder: **Hintermeier, Karl, Dr.**
**Hünfelder Strasse 18**
**D-6000 Frankfurt am Main(DE)**
Erfinder: **Müller, Manfred**
**Untere Schönau 31**
**D-6440 Gelnhausen 5(DE)**
Erfinder: **Münch, Norbert, Dr.**
**Johann-Strauss-Strasse 39**
**D-6233 Kelkheim(DE)**
Erfinder: **Wagener, Hans**
**Am Lotzenwald 8**
**D-6238 Hofheim/Taunus(DE)**

(74) Vertreter: **Urbach, Hans-Georg, Dr. et al**
**Hanauer Landstrasse 526**
**D-6000 Frankfurt am Main 61(DE)**

## (54) Methylolierte und gegebenenfalls veretherte Fluoralkylliganden enthaltende Urethane.

(57) Durch Umsetzung von Fluoralkylliganden enthaltenden Urethanen mit Formaldehyd und gegebenenfalls sich anschließender Veretherung werden methylolierte und gegebenenfalls veretherte Fluoralkylliganden enthaltende Urethane erhalten, die in Form ihrer Lösungen oder wäßrigen Dispersionen zur Behandlung von Textilien und von Leder geeignet sind.

EP 0 304 016 A1

**Methylolierte und gegebenenfalls veretherte Fluoralkylliganden enthaltende Urethane**

Die Erfindung betrifft methylolierte und gegebenenfalls veretherte Fluoralkylliganden enthaltende Urethane, Verfahren zu ihrer Herstellung, ihre wäßrigen Dispersionen und ihre Verwendung.

Fluoralkylliganden enthaltende Urethane zur Behandlung von Textilien sind bereits bekannt, vgl. z.B. US-Patentschriften 4 264 484, 4 340 749 und 4 468 527. Die erfindungsgemäßen methylolierten und gegebenenfalls veretherten Fluoralkylliganden enthaltenden Urethane sind den bisher bekannten, Fluoralkylliganden enthaltenden Verbindungen überlegen.

Die erfindungsgemäßen methylolierten und gegebenenfalls veretherten Fluoralkylliganden enthaltenden Urethane sind herstellbar durch Umsetzung eines Fluoralkylliganden enthaltenden Urethans der allgemeinen Formel 1

$$(R_f\text{-}(SO_2NR^1)_a(CH_2)_x\text{-}O\text{-}(CH_2\text{-}\underset{Y}{\underset{|}{C}}H\text{-}O)_m\text{-}CO\text{-}NH)_p\text{-}R \quad (1)$$

worin
$R_f$ eine Perfluoralkylgruppe mit 4 bis 20 C-Atomen,
$R^1$ Wasserstoff oder eine Alkylgruppe mit 1 bis 4 C-Atomen
a die Zahl 0 oder 1,
x eine Zahl von 1 bis 4,
Y Wasserstoff, $-CH_3$ oder $-CH_2Cl$,
R einen organischen Rest,
p eine Zahl von 1 bis 4,
m eine Zahl von 0 bis 4
bedeuten, mit Formaldehyd im Molverhältnis 1 : (0,5 bis 1,1 t), wobei t die Zahl der insgesamt in der Verbindung vorhandenen methylolierbaren -NH-Gruppen ist, und gegebenenfalls anschließender Veretherung mit einem niederen Alkanol mit 1 bis 4 C-Atomen oder Ethylenglykolmonoalkylether mit 1 bis 4 C-Atomen im Alkylrest.

Die Perfluoralkylgruppe $R_f$ kann geradkettig oder verzweigt sein; im Falle einer verzweigten Perfluoralkylgruppe ist die endverzweigte bevorzugt. Von den beiden Perfluoralkylgruppen, den geradkettigen oder verzweigten, sind die geradkettigen bevorzugt. Beim Perfluoralkyl-Rest handelt es sich in der Regel um ein Gemisch von Perfluoralkylgruppen mit der obengenannten Anzahl von C-Atomen. Die Perfluoralkylgruppe $R_f$ besitzt vorzugsweise 6 bis 16 C-Atome.

Für a ist die Zahl Null bevorzugt. Der für $R^1$ stehende Alkylrest kann geradkettig oder verzweigt sein. Für x ist die Zahl 2 bevorzugt.

Der für R stehende organische Rest kann ein aliphatischer, aromatischer oder araliphatischer Rest sein, der auch Heteroatome, vor allem Stickstoff und/oder Sauerstoff und insbesondere eine oder mehrere methylolierbare -NH-Gruppen enthalten kann.

Beispiele für zweiwertige aliphatische Reste R sind solche der Formeln 2, 3 und 4

$-(CH_2)_q-$ (2)
$-(CH_2)_q\text{-}O\text{-}(CH_2)_q-$ (3)
$-(CH_2)_q\text{-}NH\text{-}(CH_2)_q-$ (4)

wobei q eine Zahl von 2 bis 10, insbesondere 2 bis 8 darstellt.

Beispiele für zweiwertige aromatische Reste R sind Phenylenreste der Formel 5, insbesondere 1,3 - und 1,4-Phenylenreste der Formeln 5a und 5b, die im Kern vor allem durch Alkylreste, insbesondere Methyl, substituiert sein können:

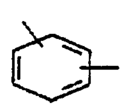
(5)

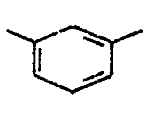
(5a)

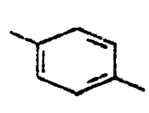
(5b)

Ein zweiwertiger araliphatischer Rest R kann auch ein solcher der Formel 6

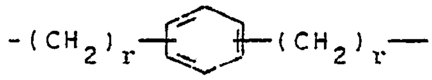 (6)

sein, wobei r eine Zahl von 1 bis 6 bedeutet und der Phenylkern auch substituiert sein kann, vor allem durch Alkylreste, insbesondere durch Methyl.

Weitere organische Reste R sind solche der nachstehenden Formeln 7 bis 12

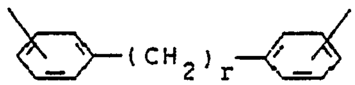 insbesondere 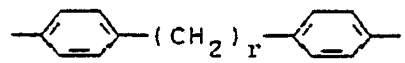

(7) (7a)

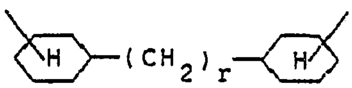 insbesondere 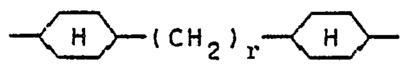

(8) (8a)

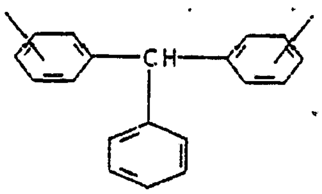 insbesondere 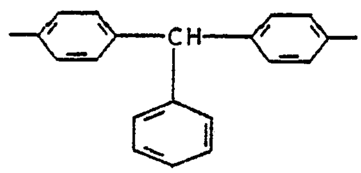

(9) (9a)

$-(CH_2)_q-NH-CO-NH-(CH_2)_q-$ (10)

$-(CH_2)_q-NH-CO-NH-(CH_2)_q-NH-CO-NH-(CH_2)_q-$ (11)

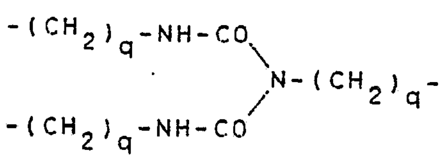 (12)

wobei in den Formeln 7, 7a, 8 und 8a r eine Zahl von 1 bis 6, vorzugsweise 1, bedeutet. In den Formeln 10 bis 12 bedeutet q eine Zahl von 2 bis 10, insbesondere 2 bis 8, vorzugsweise 6.

Weiterhin kann R ein Rest der Formel 13

$-R^2-(NH-CO-R^3)_w$ (13)

sein, wobei w eine Zahl von 1 bis 2 bedeutet, mit der Maßgabe, daß die Summe aus p + w höchstens 3 ist und $R^2$ ein (p + w)-valenter organischer Rest ist, beispielsweise einer der vorstehend für R angegebenen aliphatischen, aromatischen oder araliphatischen Reste der Formeln 2 bis 9 oder ein Rest der Formeln 10 bis 12, und $R^3$ einen Rest der Formeln 14 bis 16

$-NH-R^2-(NH-OC-(O-\underset{Y}{C}H-CH_2)_m-O-(CH_2)_x-(R^1NSO_2)_a-R_f$ (14)

$-O-(CH_2CH_2CH_2CH_2O)_sH$ (15)

$-O-(CH_2CH_2OCH_2CH_2O)_sH$ (16)

oder den Rest einer CH-aciden oder NH-aciden Verbindung, oder einen Rest der Formel 17

$-X^1-R^4$ (17)

bedeutet. In den Formeln 15 und 16 ist s eine Zahl von 1 bis 15, vorzugsweise 1 bis 10.

$R^2$ ist vorzugsweise ein Rest der Formel 18

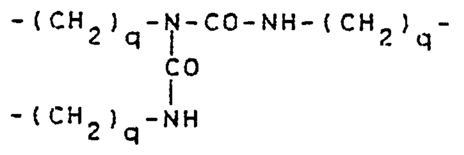

(18)

wobei q eine Zahl von 2 bis 10, insbesondere 2 bis 8, vorzugsweise 6, bedeutet.

$R^3$ stellt z.B. auch den durch Entfernung eines Wasserstoffatoms entstandenen Rest einer CH-aciden oder NH-aciden Verbindung dar. Derartige CH-acide Verbindungen sind beispielsweise Verbindungen mit einer aktiven Methylengruppe, wie z.B. Malonester, Cyanessigester, Acetylace ton, Acetessigester, Bernsteinsäureester, N-Methylpyrrolidon etc. Geeignete NH-acide Verbindungen sind z.B. Lactame, wie z.B. ε-Caprolactam, und Pyrrolidon.

In der Formel 17 bedeutet $X^1$ eine der Gruppen entsprechend den nachstehenden Formeln 19 bis 23, die mit einer Alkylgruppe mit 1 bis 4 C-Atomen ein- oder mehrfach, vorzugsweise einfach, substituiert sein können (die Formeln 19 bis 23 repräsentieren Reste von gegebenenfalls mit $C_1$-bis $C_4$-Alkylgruppen substituierten aromatischen Dihydroxy-Verbindungen, Diamino-Verbindungen und Aminohydroxy-Verbindungen, die nach Abgabe von aktiven Wasserstoffatomen an Isocyanatgruppen vorliegen):

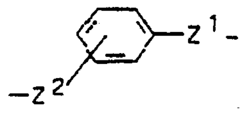 (19)

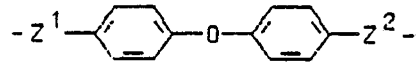

(20)

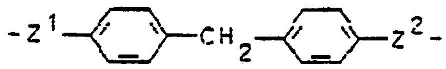

(21)

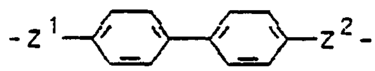

(22)

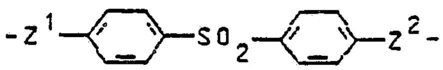

(23)

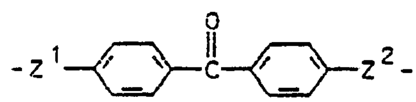

(24)

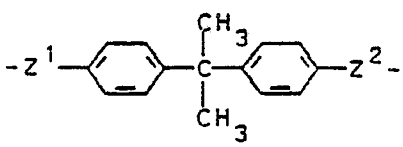

(25)

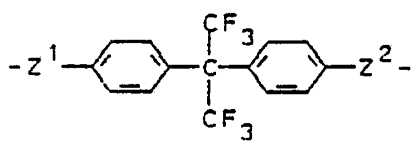

(26)

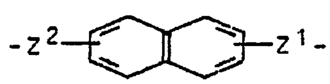 (27)

worin $Z^1$ und $Z^2$, die gleich oder verschieden sein können, für O oder NH stehen, und $R^4$ bedeutet das Wasserstoffatom, eine Alkylgruppe mit 1 bis 4 C-Atomen oder eine Gruppe entsprechend der nachstehenden Formel 28

$$(R_f\text{-}(SO_2NR^1)_a\text{-}(CH_2)_x\text{-}O\text{-}(CH_2\text{-}\underset{Y}{\underset{|}{CH}}\text{-}O)_m\text{-}CO\text{-}NH)\text{-}R^2\text{-}NH\text{-}CO\text{-} \quad (28)$$

Bevorzugte Reste R sind:

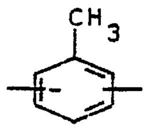 insbesondere 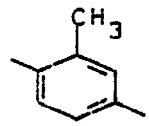

(29) (29a)

ferner:

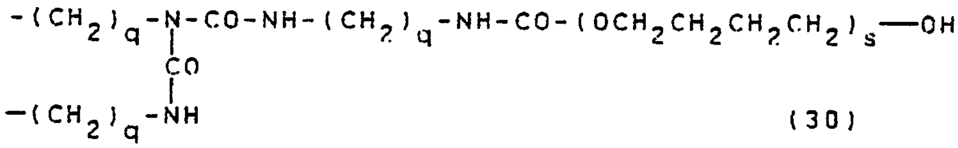

(30)

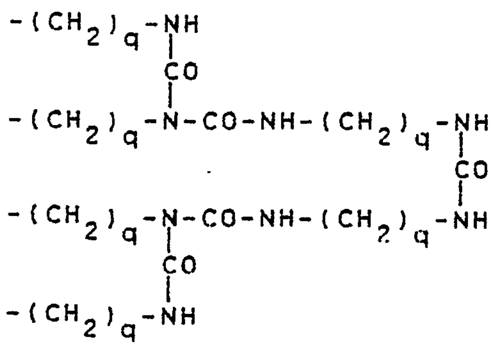

(31)

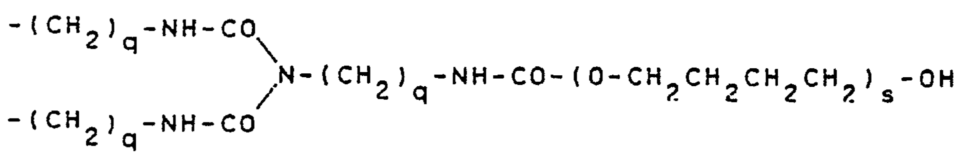

(32)

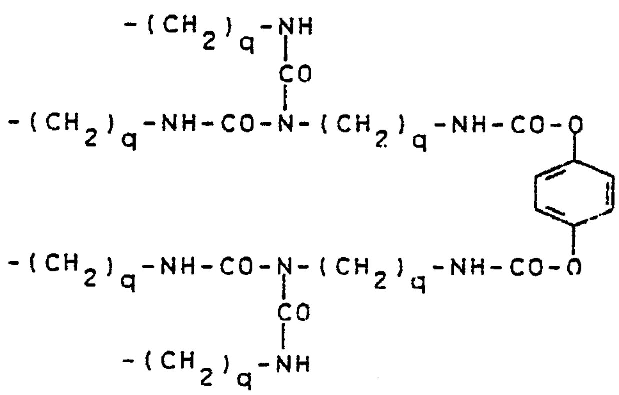

(33)

In den vorstehenden Formeln 30 bis 33 bedeutet q eine Zahl von 2 bis 10, insbesondere 2 bis 8, vorzugsweise 6, und s eine Zahl von 1 bis 15, insbesondere 1 bis 10, und an den freien Bindungen sind gleiche oder verschiedene Reste der Formel

$(R_f-(SO_2NR^1)_a-(CH_2)_x-O-(CH_2-\underset{Y}{C}H-O)_m-CO-NH)-$ (1a)

vorhanden.

Die in der Verbindung der Formel 1 vorhandenen Alkylreste können geradkettig oder verzweigt sein.

Bevorzugte Ausgangsverbindungen der Formel 1 sind solche, die für $R_f$ einen der genannten bevorzugten Perfluoralkylreste mit 6 bis 16 C-Atomen besitzen und bei denen a = 0 und x = 2 bedeuten und bei denen R eine der bevorzugten Bedeutungen der Formeln 29, 29a bis 33 besitzt, wobei in den Formeln 30 bis 33 q vorzugsweise 6 bedeutet. Y bedeutet vorzugsweise $-CH_2Cl$.

Die erfindungsgemäße Methylolierung wird so durchgeführt, daß 1 mol der Verbindung der allgemeinen Formel 1 mit 0,5 mol bis 1,1 t mol Formaldehyd, wobei t die Zahl der in der Verbindung der allgemeinen Formel 1 insgesamt vorhandenen methylolierbaren -NH-Gruppen ist, bei einer Temperatur von 20 bis 70° C, vorzugsweise 30 bis 65° C, umgesetzt wird. Die Methylolierung kann ohne Lösungsmittel durchgeführt werden, wobei dann Paraformaldehyd oder wäßriger Formaldehyd mit geschmolzenem Ausgangsprodukt der allgemeinen Formel 1 zur Umsetzung gebracht wird. Im allgemeinen wird die Methylolierung jedoch in einem Lösungsmittel durchgeführt. Geeignete Lösungsmittel sind polare organische Lösungsmittel, die gegenüber den Ausgangs- und Endverbindungen inert sind, wie z.B. Säureamide, wie Dimethylformamid; N-Methyl-pyrrolidon; Ester, wie Butylacetat; Glykolether, wie Ethylenglykoldimethylether und Diethylenglykoldimethylether, Alkohole, wie insbesondere solche mit 1 bis 4 C-Atomen, wie Methanol, Butanol, Isobutanol.

Dabei kann der Formaldehyd in Form wäßriger Lösungen eingesetzt werden, wenn diese sich mit dem Lösungsmittel mischen. Der Formaldehyd wird jedoch zweckmäßigerweise in Form einer Lösung in einem organischen Lösungsmittel eingesetzt. Geeignet sind z.B. die handelsüblichen Lösungen von Formaldehyd in Isobutanol oder Lösungen von Paraformaldehyd in einem organischen Lösungsmittel, z.B. einem Alkanol.

Das Molverhältnis zwischen der Verbindung der Formel 1 und Formaldehyd wird insbesondere so gewählt, daß es 1 : (0,5 bis (t-1)) beträgt, wobei t die Zahl der insgesamt im Molekül vorhandenen methylolierbaren -NH-Gruppen ist. Vorzugsweise beträgt bei der Methylolierung das Molverhältnis Verbindung der Formel 1 : Formaldehyd = 1 : (0,5 bis 0,525 t), wobei t die angegebene Bedeutung besitzt. Wie aus dem Vorstehenden zu entnehmen ist, wird vorzugsweise nur eine Teilmethylolierung durchgeführt.

Vorzugsweise erfolgt die Methylolierung in Anwesenheit eines alkalischen Katalysators, wofür vorzugsweise tertiäre Amine verwendet werden, so z.B. N,N-Dimethyl-ethanolamin oder Triethylendiamin (= 1,4-Diazabicyclo(2.2.2)-octan = DABCO). Im Hinblick auf eine sich anschließende Dispergierung des gegebenenfalls veretherten Methylolierungsprodukts ist es zweckmäßig, als alkalischen Katalysator ein solches tertiäres Amin zu verwenden, das bei der anschließenden Dispergierung als Emulgator wirkt. Hierfür sind beispielsweise Perfluoralkylgruppen enthaltende tertiäre Amine, wie z.B. die folgenden geeignet:

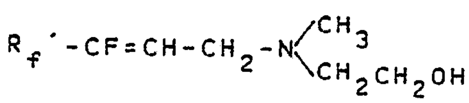 (34)

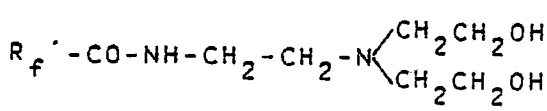 (35)

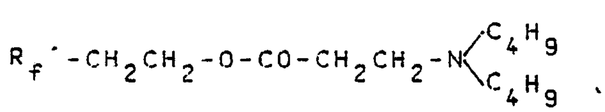 (36)

wobei $R_f'$ einen Perfluoralkylrest mit 4 bis 15 C-Atomen, vorzugsweise 6 bis 12 C-Atomen, bedeutet.

Die Methylolierung wird normalerweise in Anwesenheit von 0,01 bis 5 Gew.%, vorzugsweise 0,1 bis 1 Gew.%, eines basischen Katalysators, bezogen auf die zu methylolierende Verbindung, durchgeführt. Anstelle eines basischen Katalysators kann auch ein Gemisch aus zwei oder mehreren basischen Katalysatoren verwendet werden.

An die Methylolierung kann sich gegebenenfalls eine Veretherung mit einem niederen Alkanol, d.h.

einem Alkanol mit 1 bis 4 C-Atomen, oder einem Ethylenglykolmonoalkylether mit 1 bis 4 C-Atomen im Alkylrest anschließen. Das Alkanol und der Alkylrest in dem Ethylenglykolmonoalkylether können geradkettig oder verzweigt sein. Die gegebenenfalls durchgeführte Veretherung erfolgt in der Regel so, daß der Veretherungsalkohol zu dem Methylolierungsprodukt im Überschuß zugegeben und das Reaktionsmedium sauer gestellt wird, z.B. durch Zugabe einer Mineralsäure. Dann wird einige Stunden, in der Regel 1 1/2 bis 4 Stunden, auf Temperaturen von 40 bis 65 °C erhitzt und zum Schluß überschüssiger Veretherungsalkohol unter vermindertem Druck abdestilliert.

Die erfindungsgemäße Methylolierung kann auch zweckmäßigerweise gleichzeitig mit einer Dispergierung der zu methylolierenden Verbindung der Formel 1 durchgeführt werden. Diese Dispergierung wird in gleicher Weise durchgeführt, wie die noch zu erwähnende Dispergierung der methylolierten und gegebenenfalls veretherten Verbindungen.

Nach der erfindungsgemäßen Methylolierung und nach an sich gegebenenfalls an die Methylolierung anschließenden Veretherung sind die ursprünglich in der Verbindung der Formel 1 vorhanden gewesenen Wasserstoffatome an den methylolierbaren -NH-Gruppen ganz oder vorzugsweise zum Teil durch $-CH_2OH$- oder $-CH_2OR^5$- oder $-CH_2OCH_2CH_2OR^5$-Gruppen ersetzt, wobei $R^5$ geradkettiges oder verzweigtes Alkyl mit 1 bis 4 C-Atomen bedeutet. Vorzugsweise beträgt in den erfindungsgemäßen, methylolierten und gegebenenfalls veretherten Verbindungen das Verhältnis zwischen der Gesamtzahl t der ursprünglich vorhanden gewesenen methylolierbaren -NH-Gruppen und der Gesamtzahl der vorhandenen $-N(CH_2OH)$-Gruppen oder der vorhandenen $-N(CH_2OR^5)$- und/oder $-N(CH_2OCH_2CH_2OR^5)$-Gruppen 1 : (0,5 bis (t-1)), vorzugsweise 1 : (0,5 bis 0,525 t).

Die erfindungsgemäßen, methylolierten und gegebenenfalls veretherten, Fluoralkylliganden enthaltenden Urethane sind den entsprechenden, nicht methylolierten, Fluoralkylliganden enthaltenden Produkten bei der Hydrophob-und Oleophobbehandlung von Textilien und von Leder im Ausrüstungseffekt und in der Waschbeständigkeit überlegen.

Die erfindungsgemäßen Verbindungen sind hervorragend zur Behandlung von Textilien und von Leder geeignet und bewirken dabei insbesondere oleophobierende, hydrophobierende, schmutzabweisende und leitfähigkeitsverbessernde Effekte. Sie können in Form ihrer Lösungen in organischen Lösungsmitteln oder in Form ihrer wäßrigen Dispersionen eingesetzt werden.

Für die Anwendung der erfindungsgemäßen Verbindungen aus ihren Lösungen sind z.B. geeignete organische Lösungsmittel: Alkohole, wie z.B. Isopropanol; Ketone, wie z.B. Aceton, Methylethylketon, Methylpropylketon, Diethylketon; Ester, wie z.B. Ethylacetat, Propylacetat, Butylacetat oder Mischungen aus zwei oder mehreren organischen Lösungsmitteln. Der Wirkstoffgehalt der organischen Lösung kann in weiten Grenzen schwanken und beträgt vorzugsweise 1 bis 20 Gew%. Die organische Lösung kann auch zwei oder mehr erfindungsgemäße Wirkstoffe und gegebenenfalls noch andere Ausrüstungs- und/oder Hilfsmittel enthalten.

Vorzugsweise erfolgt die Anwendung der erfindungsgemäßen Wirkstoffe aus ihren wäßrigen Dispersionen, die aber ebenfalls bevorzugt Lösungsmittel enthalten können.

Zur Herstellung der wäßrigen Dispersionen werden die Wirkstoffe, zusammen mit kationischen oder anionischen und gegebenenfalls nichtionischen Dispergier- und/oder Emulgiermitteln und gegebenenfalls weiteren Hilfsmitteln und Lösungsmitteln in Wasser, unter Zufuhr einer verhältnismäßig großen Energiemenge, dispergiert. Als derartige Dispergier- und/oder Emulgiermittel kommen beispielsweise Verbindungen der Formeln 34 bis 36 und die noch zu nennenden kationischen, anionischen und nichtionischen Emulgier- und Dispergiermittel in Betracht. Dabei werden die Wirkstoffe zumindest in einem Teil der zur Anwendung kommenden Menge des Lösungsmittels oder Lösungsmittelgemisches vorgelegt und die Dispergierung zweckmäßigerweise in zwei Teilschritte aufgeteilt, wobei zuerst eine Vordispergierung und anschließend eine Feindispergierung vorgenommen wird. Auch die Vordispergierung wird zweckmäßigerweise durch Anwendung hoher Scherkräfte, beispielsweise durch die Verwendung eines schnell laufenden Rüh rers, wie z.B. bei einer Dispergiermaschine von Typ Ultraturrax, genommen, und die dabei erhaltene Vordispersion wird anschließend z.B. einer Ultraschallbehandlung oder einer Behandlung in einem Hochdruckhomogenisator unterzogen. Nach der Beendigung dieser Behandlung liegt die Teilchengröße in der Dispersion zu über 80%, vorzugsweise zu über 95 %, bei oder unter 1 µm.

In der Regel enthält die fertige wäßrige Dispersion 5 bis 30 Gew.% erfindungsgemäßen Wirkstoff, 2 bis 15 Gew.% Dispergier-und/oder Emulgiermittel, 5 bis 30 Gew.% eines Lösungsmittels oder Lösungsmittelgemisches, der Rest ist Wasser. Selbstverständlich können anstelle eines erfindungsgemäßen Wirkstoffes auch ein Gemisch von erfindungsgemäßen Wirkstoffen, und anstelle eines Dispergier-und/oder Emulgiermittels kann auch ein Gemisch derartiger Mittel bei der Dispergierung zur Anwendung kommen.

Als Lösungsmittelzusatz für die Dispergierung werden gewöhnlich Gemische aus wasserlöslichen und wasserunlöslichen Lösungsmitteln verwendet, wobei die ersteren meist überwiegen. Geeignete wasserlösliche Lösungsmittel, sind z.B. Mono- oder Di-Alkohole, niedere Ketone, Polyglykolester und Polyglykolether oder Gemische derartiger Lösungsmittel. Vorteilhafterweise enthält die Lösungsmittelkomponente mindestens ein hochsiedendes, wasserlösliches Lösungsmittel, d.h. ein Lösungsmittel, dessen Siedepunkt über ca. 150 °C liegt. Geeignete wasserunlösliche Lösungsmittel sind z.B. Ester, Ether und/oder höhere Ketone. Niedrigsiedende Lösungsmittelanteile können gegebenfalls zu einem späteren Zeitpunkt wieder entfernt, z.B. abdestilliert werden.

Als geeignete wasserlösliche, hochsiedende Lösungsmittel kommen insbesondere die ($C_1$-$C_4$)-Monoalkyl- und -Dialkyl ether des Diethylenglykols und/oder Dipropylenglykols in Betracht. Günstig für die Stabilität der Dispersion ist ferner ein Zusatz von Isopropanol, Glykol oder Glyzerin, einzeln oder im Gemisch, vorzugsweise in einer Menge von 1 bis 5 Gew.%, bezogen auf die Endeinstellung. Als geeignete wasserunlösliche Lösungsmittel kommen vor allem Butylester in Frage, insbesondere Butylacetat.

Besonders günstige Effekte auf Teppichen, insbesondere im Hinblick auf die schmutzabweisende Wirkung, werden auch erhalten, wenn die erfindungsgemäße wäßrige Dispersion zusätzlich mindestens ein dispergiertes, gegebenenfalls auch fluorhaltiges (Meth)Acrylsäureester-polymerisat oder -copolymerisat in Mengen von 5 bis 25 Gew.% enthält, wobei diese Menge so gewählt ist, daß die Menge des (Meth)-Acrylsäureester-polymerisats oder -copolymerisats zusammen mit der Wirkstoffmenge 15 bis 30 Gew.% beträgt. Derartige (Meth)Acrylsäureester-polymerisate bzw. -copolymerisate werden zweckmäßigerweise in Form einer getrennt hergestellten wäßrigen Dispersion den erfindungsgemäßen Dispersionen zugesetzt.

Die (Meth)Acrylsäureester-polymerisate bzw. -copolymerisate enthalten normalerweise Bausteine von Estern der Acryl- und/oder Methacrylsäure mit $C_1$- bis $C_{18}$-Alkoholen und können z.B. in an sich bekannter Weise hergestellt werden. Methacrylester-copolymerisate sind bevorzugt, insbesondere dann, wenn das zu ihrer Herstellung benutzte Monomerengemisch mindestens 80 Gew.% Ester von $C_1$- bis $C_4$-Alkoholen enthält. Bevorzugt sind Copolymerisate aus Methacrylsäuremethylester und Methacrylsäureisobutylester, insbesondere dann, wenn in dem Copolymerisat der Methylesteranteil überwiegt. Besonders bevorzugt ist ein Copolymerisat, hergestellt aus Methacrylsäuremethylester und Isobutylester im Gewichtsverhltnis 3 : 1. Die Her stellung eines Methacrylsäureester- Copolymerisats und seine Dispergierung ist in den Beispielen 3 b) und 3 c) beschrieben. Andere (Meth)Acrylsäureester-polymerisate bzw. -copolymerisate können analog hergestellt und dispergiert werden.

Das (Meth)Acrylsäureester-polymerisat bzw. -copolymerisat wird kationisch dispergiert, wenn es einer kationischen Dispersion der erfindungsgemäßen methylolierten und gegebenenfalls veretherten Wirkstoffe zugesetzt wird, und es wird anionisch dispergiert, wenn es einer anionischen Dispersion der erfindungsgemäßen Wirkstoffe zugesetzt wird.

Geeignete kationische Dispergier- und/oder Emulgiermittel für die Herstellung der erfindungsgemäßen wäßrigen kationischen Dispersionen bzw. für die Dispergierung des (Meth)Acrylsäureesterpolymerisats sind z.B. handelsübliche technische Produkte der Formeln 37 bis 41.

$$(R_f'\text{-CF=CH-CH}_2\text{-NR}^6R^7)^{\oplus}Z^{\ominus} \quad (37)$$

$$(R_f'\text{-CF=CH-CH}_2\text{-N(CH}_3)_2\text{CH}_2\text{CH}_2\text{OH})^{\oplus}Z^{\ominus} \quad (38)$$

$$R_f'\text{-CF=CH-CH}_2\text{-NR}^8\text{-CH}_2\text{CH}_2\text{OH})^{\oplus}Z^{\ominus} \quad (39)$$

wobei
$R_f'$ einen Perfluoralkylrest mit 4 bis 15 C-Atomen, vorzugsweise 6 bis 12 C-Atomen,
$R^6$, $R^7$ = $-CH_3$, $-C_2H_5$, $-C_3H_7$ oder $-C_4H_9$,
$Z^{\ominus}$ = $SO_4CH_3$, Cl,
$R^8$ = -H oder $-CH_3$
bedeuten. Derartige kationische Dispergier- und/oder Emulgiermittel können, wie auch die noch zu nennenden anionischen Dispergier- und/oder Emulgiermittel, auch in Kombination mit nichtionischen Dispergier- und/oder Emulgiermitteln zur Anwendung kommen. Für eine derartige Kombination geeignete nichtionische Produkte sind z.B. Polyoxyethylenderivate von Sorbitanhydriden, die teilweise mit Fettsäure verestert sind, wie z.B. Polyoxyethylensorbitanmonooleat, ferner Polyoxyethylenderivate von Fettalkoholen oder von Fluoralkoholen. Geeignete Polyoxyethylenderivate von Fluoralkoholen besitzen die Formel 40

$$R_f'\text{-CH}_2\text{CH}_2\text{-(OCH}_2\text{CH}_2)_u\text{-OH} \quad (40)$$

wobei u eine Zahl von 4 bis 16, vorzugsweise 5 bis 13 ist und $R_f'$ die bereits angegebene Bedeutung besitzt. Auch derartige nichtionische Produkte sind handelsüblich.

Die anionischen wäßrigen Dispersionen der erfindungsgemäßen methylolierten und gegebenenfalls veretherten Wirkstoffe sind bevorzugt. Zur Herstellung dieser anionischen wäßrigen Dispersionen bzw. des anionisch dispergierten (Meth)Acrylsäurepolymerisats oder -copolymerisats wird vorzugsweise mindestens ein Emulgator der Formel 41

$R_f'-CH_2CH_2O-CO-NH-CH_2CH_2-SSO_3X^2$ (41)

wobei $R_f'$ die bereits genannte Bedeutung besitzt und $X^2$ ein einwertiges Kation bedeutet, in Kombination mit mindestens einem nichtionischen Emulgator der Formel 42

$R_f'-CH_2CH_2-(OCH_2CH_2)_v-OH$ (42)

wobei $R_f'$ die bereits genannte Bedeutung besitzt und v ein Zahl von 0 bis 10 bedeutet, verwendet.

Falls mehrere Verbindungen mit $R_f'$-Resten zum Einsatz kommen, z.B. solche der Formeln 34 bis 42, brauchen die $R_f'$-Reste nicht identisch zu sein.

Die Verbindungen der Formel 34 bis 42 können in Form ihrer handelsüblichen technischen Gemische eingesetzt werden, die in der Regel mehrere Verbindungen der genannten Art mit verschiedenen Perfluoralkylresten $R_f'$ enthalten.

Das einwertige Kation $X^2$ in den Verbindungen der Formel 41 stellt in der Regel ein Alkalimetallkation, insbesondere das Natrium- oder Kaliumkation oder das Ammoniumkation dar. Das Ammoniumkation kann gegebenenfalls auch durch organische Reste substituiert sein, beispielsweise Triethanolammonium darstellen.

Bei den handelsüblichen Gemischen der Emulgatoren der Formel 40 beträgt v insbesondere ca. 6.

Die erfindungsgemäßen methylolierten und gegebenenfalls veretherten Verbindungen der Formel 1 lassen sich leichter in eine wäßrige Dispersion, insbesondere eine anionische wäßrige Dispersion überführen, als die nichtmethylolierten Ausgangsverbindungen der Formel 1.

Die erfindungsgemäßen Wirkstoffe sind in Form ihrer Lösungen in organischen Lösungsmitteln und vorzugsweise in Form ihrer wäßrigen Dispersionen zur Behandlung von Textilien aus natürlichen oder synthetischen Fasern, insbesondere aus Polyamid, Polyester, Polyacrylnitril und Wolle oder Gemischen dieser Faserarten, sowie für Leder geeignet. Das Textilmaterial kann in beliebiger Form vorliegen, so z.B. als Faden, Faser, Garn, Flocke, als Gewebe, Gestrick oder Vlies, insbesondere jedoch als Teppich. Die erfindungsgemäßen Wirkstoffe können sowohl allein für die Behandlung eingesetzt werden, wie auch in Kombination mit anderen Behandlungsmitteln, wie Textilharzen auf der Basis Glyoxal oder deren Derivaten, Weichmachern, PVA und EVA oder ähnlichen Dispersionen.

Die erfindungsgemäßen wäßrigen Dispersionen erfüllen alle Forderungen der Praxis und zeigen insbesondere eine hervorragende Langzeitstabilität bei Temperaturen von -20 bis +40° C. Sie gefrieren zwar bei Minustemperaturen, die Dispersion bleibt aber nach dem Auftauen erhalten, im Gegensatz zu den bisher bekannten Dispersionen. Die erfindungsgemäßen wäßrigen, insbesondere anionischen Dispersionen zeigen bei der Textilbehandlung einen hervorragenden oleophobierenden, hydrophobierenden, schmutzabweisenden und leitfähigkeitsverbessernden Effekt. Die Haftung der Wirkstoffe auf dem Substrat ist auch bei Wasch- oder Reinigungsvorgängen gut.

Die erfindungsgemäßen Wirkstoffe können in Form ihrer Lösungen in organischen Lösungsmitteln oder vorzugsweise in Form ihrer wäßrigen Disperisonen auf das Textilmaterial oder Leder aufgebracht werden. Dabei können die Lösungen bzw. Dispersionen in der Form verwendet werden, in der sie bei der Herstellung anfallen. Normalerweise wird man sie jedoch zur Anwendung mit Wasser auf einen Feststoffgehalt von 1 bis 10 Gew.%, vorzugsweise von 1,5 bis 5 Gew.%, verdünnen. Die Aufbringung auf das zu behandelnde Textilmaterial kann in jeder geeigneten Weise erfolgen, so z.B. durch Sprühen, Pflatschen, Foulardieren etc. Eine Wiederholung des Prozesses kann die Durchdringung des Substrats fördern und die Effektivität des Wirkstoffs erhöhen. Die Auftragsmenge wird so gewählt, daß auf dem Material normalerweise 0,01 bis 1 Gew.%, vorzugsweise 0,05 bis 0,2 Gew.%, Fluor vorhanden sind. Dies entspricht etwa einer Menge von 0,1 bis 10, vorzugsweise 0,5 bis 4 Gew.% Feststoffgehalt. Nach dem Aufbringen auf das zu behandelnde Material erfolgt eine Trocknung bei Temperaturen bis ca. 120° C, z.B. bei 100 bis 120° C, und anschließend wird eine Wärmebehandlung bei Temperaturen von ca. 130 bis 200° C je nach Art des Substrats, vorzugsweise bei 140 bis 180° C, durchgeführt, die normalerweise etwa 4 min bis etwa 30 s dauert. Die Trocknung und die Wärmebehandlung können in verschiedenen Vorrichtungen oder in der gleichen Vorrichtung durchgeführt werden.

Es hat den Anschein, als ob die in der Dispersion vorzugsweise enthaltenen, hochsiedenden organischen Lösungsmittel auch eine wichtige Bedeutung bei der Fixierung des Wirkstoffs auf der Faser im Sinne

einer Art Carrier-Effekt besitzen.

Die Ausgangsverbindungen der Formel 1 sind zum Teil bekannt. Die bekannten und die noch nicht beschriebenen Ausgangsverbindungen der Formel 1 können durch Umsetzung eines Fluoralkanols der Formel 1 b

$$(R_f\text{-}(SO_2NR^1)_a\text{-}(CH_2)_x\text{-}O\text{-}(CH_2\text{-}\underset{\displaystyle Y}{\underset{|}{C}}H\text{-}O)_m\text{-}H \qquad (1\ b)$$

mit einem Isocyanat der Formel 43

$$(OCN)_p\text{-}R \qquad (43)$$

wobei $R_f$, $R^1$, Y, a, x, m, p und R die bereits genannten Bedeutungen besitzen, hergestellt werden. Die Umsetzung erfolgt normalerweise im Molverhältnis (1 b) : (43) = p : 1. Sie kann jedoch auch mit einem Unterschuß der Verbin dung 1 b, insbesondere im Molverhältnis (1 b) : (43) = (p - 1) : 1 durchgeführt werden, wenn eine (oder gegebenenfalls auch mehrere) in dem Polyisocyanat der Formel 45 vorhandene Isocyanatgruppe(n) durch anschließende Umsetzung, z.B. mit einem Alkohol, einer CH-aciden oder NH-aciden Verbindung, in einen oder mehrere von 1a abweichenden Substituenten umgewandelt werden soll-(en).

Bei der Umsetzung des Isocyanats der Formel 43 mit einem Unterschuß eines Fluoralkanols der Formel 1 b entsteht zunächst das Addukt der Formel 44

$$(R_f\text{-}(SO_2NR^1)_a\text{-}(CH_2)_x\text{-}O\text{-}(CH_2\text{-}\underset{\displaystyle Y}{\underset{|}{C}}H\text{-}O)_m\text{-}CO\text{-}NH)_p\text{-}R^1\text{-}(NCO)_w \qquad (44)$$

das dann mit einem anderen Fluoralkanol der Formel 1 b oder einer CH-aciden oder NH-aciden Verbindung oder einer Verbindung der Formel 45

$$H\text{-}R^3 \qquad (45)$$

worin $R^3$ die bereits eingangs genannte Bedeutung besitzt, oder mit Wasser umgesetzt wird.

Fluoralkanole der Formel 1 b sind für m = 0 handelsüblich. Fluoralkanole der Formel 1 b mit m = 1, 2, 3 oder 4 können dadurch hergestellt werden, daß man ein Perfluoralkanol der nachstehenden Formel 46

$$(R_f\text{-}(SO_2NR^1)_a\text{-}(CH_2)_x\text{-}OH \qquad (46)$$

worin $R_f$, $R^1$, a und x die bereits genannten Bedeutungen haben, oder ein Gemisch von solchen Perfluoralkylethanolen mit Epichlorhydrin für die Einführung von Y = $-CH_2Cl$, oder mit Ethylenoxid für die Einführung von Y = H, oder mit Propylenoxid für die Einführung von Y = $-CH_3$ in Gegenwart von Lewis-Säuren als Katalysator bei einer Temperatur von 50 bis 150° C, vorzugsweise 70 bis 90° C, im entsprechenden Molverhältnis umsetzt.

Bei dem Perfluoralkanol der Formel 46 handelt es sich in der Regel um preisgünstige, im Handel erhältliche Gemische mit verschiedenen Perfluoralkylresten $R_f$, in denen $R_f$ im Mittel 9 bis 13 C-Atome beträgt. Die Art der Lewis-Säure ist nicht kritisch. Bevorzugt sind $BF_3$, Bortrifluoriddiethyletherat, $SnCl_4$, $SbCl_5$. $TiCl_4$, $FeCl_3$, $PF_3$ und/oder Dibutylzinndilaurat, wobei Bortrifluoriddiethyletherat besonders bevorzugt ist. Die Menge an Katalysator beträgt im allgemeinen 0,01 bis 5 Gew.%, vorzugsweise 0,1 bis 1 Gew.%, bezogen auf Perfluoralkanol. Die Umsetzung wird vorzugsweise unter Rühren und bei dem sich einstellenden Druck durchgeführt, wobei das bevorzugt verwendete flüssige Epichlorhydrin (Siedepunkt bei Normalbedingungen 116° C) zum vorgelegten Alkohol dazugegeben wird. Die Umsetzungsdauer liegt im Bereich von etwa 0,5 bis 7 h. Es kann zweckmäßig sein, ein Lösungsmittel einzusetzen. Bevorzugte Lösungsmittel sind halogenierte Kohlenwasserstoffe, wie Tetrachlorkohlenstoff, Trichlorethylen, 1,2-Dichlorethan, Trichlorethan, Pentafluormonochlorethan und Trifluordichlorethan; Ketone, wie Methylethylketon und Cyclohexanon; Ether, wie Disopropylether und Tetrahydrofuran; Dimethylformamid und N-Methylpyrrolidon.

Die Umsetzung zwischen der Verbindung 46 und dem bevorzugt verwendeten Epichlorhydrin läuft quantitativ ab. Im erhaltenen Reaktionsprodukt wird das gegebenenfalls verwendete Lösungsmittel abdestilliert, wobei auch gegebenenfalls vorhandene flüchtige Anteile, wie nicht umge setztes Epichlorhydrin, entfernt werden. Aus Zweckmäßigkeitsgründen kann man die Destillation auch unter Vakuum (Wasserstrahlvakuum) durchführen. Die als Katalysator eingesetzte Lewis-Säure, die bei der nachfolgenden

Umsetzung mit dem Isocyanat an sich nicht stört, kann mit Hilfe von alkalischen Mitteln, vorzugsweise mit Hilfe einer wäßrigen Natriumbicarbonat-Lösung, oder einem Amin, wie Triethylamin, ausgewaschen bzw. neutralisiert werden.

Die Umsetzung zur Herstellung der Ausgangsverbindungen der Formel 1 wird in der Regel so durchgeführt, daß die Verbindungen 1 b oder das Gemisch der Verbindungen 1 b aufgeschmolzen und anschließend bei einer ca. 5 bis 10° C über dem Schmelzpunkt liegenden Temperatur das Isocyanat der Formel 43 oder das Gemisch der Isocyanate der Formel 43 unter Rühren zugetropft wird. Anschließend wird im Verlauf von ca. einer Stunde auf Temperaturen von ca. 130° C aufgeheizt, wobei die Reaktion ab einer Temperatur von ca. 80° C leicht exotherm verläuft. Abschließend wird die Umsetzung durch eine ca. dreistündige Reaktionszeit bei ca. 130° C zum Abschluß gebracht. Das Fortschreiten der Reaktion kann laufend IR-spektroskopisch an entnommenen Proben auf das Verschwinden der Isocyanatbanden kontrolliert werden. Falls die Reaktion in der angegebenen Zeit noch nicht zum Abschluß gekommen ist, muß die Reaktionszeit, z.B. auf 6 h, verlängert werden.

Zur Einführung des Restes 29 und 29a wird Tolylen-di-isocyanat, insbesonderes das 2,4- und/oder das 2,6-Tolylen-di-isocyanat, insbesondere in Form eines Handelsprodukts, verwendet, das ca. 80 Gew.% 2,4-Tolylen-di-isocyanat und ca. 20 Gew.% 2,6-Tolylen-di-isocyanat enthält.

Zur Herstellung von Ausgangsverbindungen der Formel 1, bei denen R ein Gemisch der Formeln 30 und 32 bedeutet, wird als Isocyanat der Formel 43 z.B. technisch erhältliches "Biuret-Triisocyanat" der Formel 47

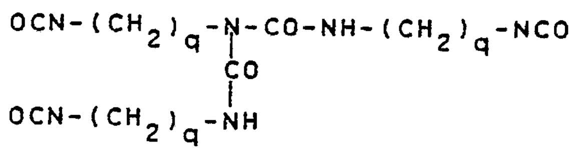

(47)

eingesetzt, wobei q die bereits angegebene Bedeutung besitzt und vorzugsweise 6 bedeutet, und mit 2 mol des Fluoralkanols der Formel 1 b und mit 1 mol einer Verbindung der Formel 48

$H-(O-CH_2CH_2CH_2CH_2)_u-OH$ (48)

umgesetzt.

Ausgangsverbindungen der Formel 1 mit einem Rest der Formel 31 lassen sich durch Umsetzung von 1 mol "Biuret-Trisiocyanat" mit 2 mol Fluoralkanol der Formel 1 b und gleichzeitige oder anschließende Umsetzung mit 0,5 mol Wasser herstellen.

Die Herstellung von typischen Ausgangsverbindungen der Formel 1 ist auch in den nachfolgenden Beispielen angegeben. Andere Ausgangsverbindungen der Formel 1 können sinngemäß analog hergestellt werden.

In den nachfolgenden Beispielen sind Temperaturen in Grad Celsius angegeben, und Prozentangaben bedeuten, sofern nichts anderes angegeben ist, Gewichtsprozente. In den Beispielen bedeuten ferner:

1. Desmodur® N 75 : ein technisches Gemisch von "Biuret-Triisocyanat" der Formel 47, worin q die Zahl 6 bedeutet, ca 75%ig in Ethylglykolacetat/Xylol (1:1), der Fa. BAYER AG.
2. "Perfluoroctylethanol" : ein technisches Gemisch verschiedener Perfluoralkylethanole der Formel

$R_F-CH_2CH_2OH$, (49)

wobei der Rest $R_F$ zu 50 bis 55% aus $C_8F_{17}$-Resten, zu 25 bis 30% aus $C_{10}F_{21}$-Resten und zu 10 bis 15% aus $C_{12}F_{25}$-Resten oder Perfluoralkyl-Resten mit mehr als 12 C-Atomen besteht und die Differenz zu 100% aus $C_6F_{13}$-Resten besteht. Das durchschnittliche Molekulargewicht beträgt 534.

3. Tween® 80 : Polyoxyethylensorbitanmonooleat mit insgesamt ca. 20 Oxyethylengruppen. (Das Warenzeichen ist für die ICI America Inc. eingetragen.)
4. DABCO : 1,4-Diazabicyclo(2.2.2)-octan( = Triethylendiamin)

Beispiel 1:

Monomethylolierung von 2,4(2,6)-Bis(2-perfluoralkylethoxycarbonylamino)-toluol

a) Herstellung des Ausgangsprodukts 2,4(2,6)-Bis(2-perfluoralkylethoxycarbonylamino)-toluol der Formel 50:

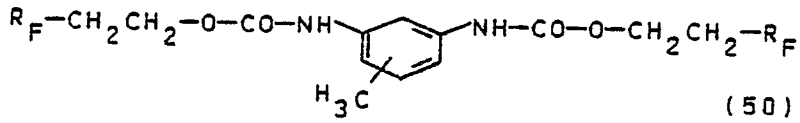

(50)

worin $R_F$ Perfluoralkyl mit 8 bis 12 C-Atomen bedeutet: 1080 g (= 2 mol) eines handelsüblichen Gemisches von 2-Perfluoralkyl-ethanolen der Formel 51

$R_F$-$CH_2CH_2$-OH (51),

in dem Einzelprodukte mit Perfluoralkylresten mit 8 bis 12 C-Atomen enthalten sind, werden in einem Reaktionsgefäß über den bei 65 ° C liegenden Schmelzpunkt aufgeheizt. Bei einer Temperatur von 70 bis 75 ° C werden dann im Verlauf von 30 min 174,16 g (= 1 mol) eines technischen Gemisches aus 80 % 2,4- und 20 % 2,6-Tolylen-di-isocyanat unter Rühren zugetropft. Anschließend wird im Verlauf von 1 h auf 130 ° C aufgeheizt, wobei die Reaktion ab 80 ° C leicht exotherm verläuft, und die Temperatur ca. 3 h bis 130 ° C gehalten.

Das Fortschreiten der Reaktion wird an entnommenen Proben IR-spektroskopisch auf das Verschwinden der Isocyanatbanden kontrolliert. Gegebenenfalls wird die Reaktionszeit verkürzt oder verlängert.

Es werden 1154 g einer gelblichen Schmelze erhalten, die beim Erkalten zu einem Kristallkuchen erstarrt.

Fp: 116 bis 120 ° C. Durchschnittlicher Fluor-Gehalt: 60,5 %.

b) Monomethylolierung des vorstehend nach a) erhaltenen Produkts, wobei ein erfindungsgemäßes Produkt der Formel 50 erhalten wird, wobei jedoch ein Wasserstoff an einer -NH-Gruppe durch -$CH_2OH$ ersetzt ist:

In einem 2 l-Schliffvierhalskolben mit Rührer, Thermometer und Rückflußkühler werden 615 g (0,5 mol) 2,4-Bis(2-perfluoralkylethoxycarbonylamino)-toluol der Formel 50, 260 g Diethylenglykoldimethylether als Lösungsmittel und 0,5 g Triethylendiamin (= "DABCO") als Katalysator bei 60-65 ° C verrührt. Dann läßt man 35 g (0,525 mol) einer 45%igen Lösung von Formaldehyd in Isobutanol zulaufen und hält dann 8 h unter Rühren bei 60-65 ° C. Man erhält 910 g Ausbeute an einer hellen, homogenen Lösung, die beim Erkalten zu einer gelblichen Paste erstarrt. Freier Formaldehyd ist praktisch nicht mehr nachzuweisen.

## Beispiel 2:

Herstellung der Bismethylolverbindung der Formel 52

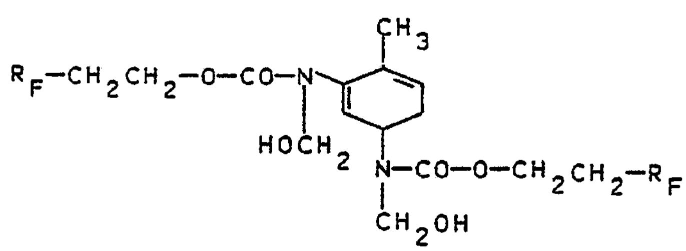

(52)

in Form eines Gemisches mit Perfluoralkylresten $R_F$ mit 8 bis 12 C-Atomen:

Das nach Beispiel 1a) erhaltene Produktgemisch der Formel 50 wird gemäß Beispiel 1 b) umgesetzt. Dabei verwendet man jedoch als basischen Katalysator anstelle von Triethylendiamin 2,5 g N,N-Dimethylethanolamin und die doppelte Menge (= 70 g) der 45%igen Lösung von Formaldehyd in Isobutanol (= 1,05 ml).

Ausbeute: 947,5 g helle Paste.

Beispiel 3:

a) Herstellung einer erfindungsgemäßen anionischen wäßrigen Dispersion des Methylolierungsproduktes gemäß Beispiel 2:

In einem 200 ml-Schliff-Dreihalskolben in Becherform werden 13,7 g des nach Beispiel 2 hergestellten Produkts, 5 g Diethylenglykol-dimethylether, 5 g Dipropylenglykolmonomethylether und 3 g technisches Perfluoralkylethylpolyglykol der Formel 53

$CF_3(CF_2)_{7-11}-CH_2CH_2-(OCH_2CH_2)_6-OH$ (53)

bei 70 bis 80°C gelöst. Dann werden bei 70°C 5 g einer 25%igen isopropanolischen Lösung eines handelsüblichen Emulgators der Formel 54

$CF_3(CF_2)_5-CH_2CH_2-O-CO-NH-CH_2CH_2-SSO_3Na$ (54)

zugemischt.

In die Lösung wird bei 70°C unter der Anwendung von starken Scherkräften einer Dispergiermaschine vom Typ Ultraturrax im Laufe von 2 bis 3 min die Lösung von 1,25 g der Verbindung der Formel 54 in 48,75 ml Wasser eingetropft, wobei die Temperatur auf 45 bis 50°C abfällt. Bei dieser Temperatur wird noch 10 bis 15 min weiterdispergiert.

Hierbei entsteht bereits eine äußerlich ansprechende Rohdispersion, die aber in dieser Form noch nicht lagerbeständig ist, sondern sich bald absetzt.

Die erhaltene Rohdispersion wird dann einer abschließenden Feinbehandlung unterworfen, und zwar durch Beschallung mittels einer Ultraschallmaschine (z.B. vom Typ Sonifier der Firma Branson), bis 90 % der Teilchen eine mittlere Größe von 1 µm erreicht oder unterschritten haben. Dies dauert gewöhnlich 10 bis 15 min. Dabei wird die Temperatur zunächst durch Wasserkühlung bei 40 bis 45°C gehalten, gegen Ende durch Kühlung mit Eiswasser auf 20 bis 30°C gesenkt.

Zu dieser so erhaltenen Feindispersion werden dann 20 g des ca. 40%igen anionisch dispergierten Methacrylestercopolymerisats gemäß dem nachfolgenden Beispiel b) hinzugefügt. Die gesamte Formulierung wird dann nochmals ca. 2 min unter Kühlung bei 20 bis 30°C mit Ultraschall behandelt. Man erhält 100 g einer feinen, milchig opaken Dispersion mit einem Fluorgehalt von 7 % (bezogen auf Wirksubstanz), die auch bei Temperaturen von -20°C bis +45°C sehr gut lagerstabil ist.

Nach einem 24stündigen Abkühlen auf -20°C konnte beim Wiederauftauen keine Veränderung der Dispersion beobachtet werden.

b) Herstellung eines Methacrylsäureester-Copolymerisats.

In einem mit Rührer und Bodenauslauf versehenen 250 ml-Vorratsgefäß werden 75 g Methacrylsäuremethylester und 25 g Methacrylsäure-isobutylester vorgelegt und zu einer homogenen Lösung verrührt, worauf der Rührer abgestellt wird.

In einem 500 ml-Polymerisationskolben, ausgerüstet mit Rührer, Thermometer, Gaseinleitungsrohr, Rückflußkühler, Tropftrichter und Zulaufmöglichkeit aus dem Vorratsgefäß werden 130 g Wasser, 30 g einer 25%igen isopropanolischen Lösung eines handelsüblichen Emulgators der Formel 54 und 22 ml der Monomerenlösung aus dem Vorratsgefäß vorgelegt. Unter Rühren und Einleiten eines schwachen Stickstoffstroms in die Lösung wird der Polymerisationskolben mittels eines elektrisch beheizten Wasserbads auf 50°C angeheizt (55°C Bad). Nach Erreichen dieser Temperatur werden 0,2 g Kaliumpersulfat in einer Portion zugegeben. Die Polymerisationsreaktion wird unmittelbar daraufhin einsetzen, was an einem Temperaturanstieg bis auf ca. 57°C sowie einer Farbänderung (bläulich fluoreszierend) festzustellen ist. Beim Überschreiten der Innentemperatur von 56°C wird mit dem Zutropfen der Monomerenlösung aus dem Vorratsgefäß sowie einer gesondert hergestellten Katalysatorlösung, bestehend aus 0,2 g Natriumpyrosulfit ($Na_2S_2O_5$) und 10 g Wasser, begonnen. Gleichzeitig kann das Einleiten von Stickstoff abgebrochen werden. Die Monomerenlösung soll nach ca. 1 h, die Katalysatorlösung etwas später zudosiert sein.

Während dieser gesamten Zutropfphase liegt die Reaktionstemperatur bei 55 bis 60°C bei unveränderter Badtemperatur von 55 °C. Nach beendetem Zutropfen wird die Polymeremulsion auf 60 bis 62 °C (65 °C Badtemperatur) angeheizt und 1 h unter diesen Bedingungen nachgerührt, anschließend auf Raumtemperatur abgekühlt und über einen PE-Siebbeutel (105 µm) filtriert. Es werden 270,4 g einer ca. 40%igen weißlichen, opaken Dispersion erhalten.

c) Herstellung eines Methacrylsäureester-Copolymerisats.

Es wird wie im vorstehenden Beispiel b) gearbeitet, jedoch werden die 30 g der isopropanolischen Lösung des dort benutzten Emulgators durch 4 g eines handelsüblichen, als Waschrohstoff benutzten Alkansulfonats (z.B. Handelsprodukt ®Warolat U der Firma Bayer AG) ersetzt.

Es werden 244,2 g einer ca. 40%igen weißlichen, opaken Dispersion erhalten.

Beispiel 4:

Monomethylolierung eines Produktgemisches der Formel 55

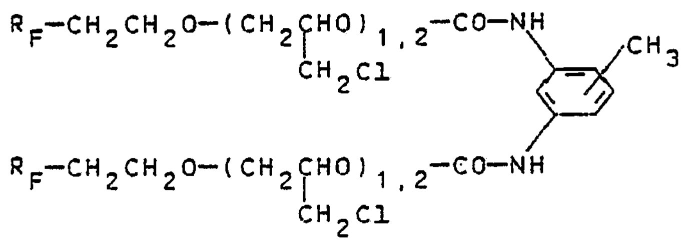

(55)

mit $R_F$ = Perfluoralkyl mit 6 bis 14 C-Atomen:

a) Herstellung des Ausgangsproduktes der Formel 55:

In einem 1 Liter-Kolben mit Rührer, Kühler mit Trockenrohr, Thermometer und Heizbad werden 548,5 g (0,84 mol) Perfluoralkylethanol-Epichlorhydrin-Addukt gemäß nachfolgendem Beispiel b) bei 80 °C geschmolzen vorgelegt. Dazu werden unter Rühren 73,1 g (= 0,42 mol) eines technischen Gemisches aus 80 % 2,4- und 20 % 2,6-Tolylen-di-isocyanat zugetropft. Anschließend wird 5 h lang bei 110 °C nachgerührt.

Ausbeute: 614 g (= 98,8 % d. Theorie) eines festen wachsartigen gelbgefärbten Produkts der Formel 55 (prakti sches Molekulargewicht = 1610) mit einem Fluorgehalt von 51,4 %.

b) Herstellung des Perfluoralkylethanol-Epichlorhydrin-Adduktes der Formel 56

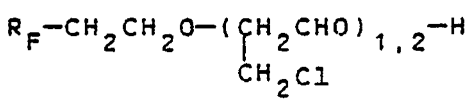

(56)

mit $R_F$ = Perfluoralkyl mit 6 bis 14 C-Atomen: In einem 2 l-Kolben mit Rührer, Rückflußkühler, Thermometer, Tropftrichter und Heizbad werden 1,5 kg (2,78 mol) eines im Handel erhältlichen Perfluoralkylethanol-Gemisches $R_FCH_2CH_2OH$ (Perfluoralkyl $R_F = C_6F_{13}$ bis $C_{14}F_{29}$; OH-Zahl = 104), 450 ml 1,2,2-Trfluortrichlorethan ($CFCl_2$-$CF_2Cl$; Kp = 48 °C) als Lösungsmittel und 15 g Bortrifluoriddiethyletherat als Katalysator (das ist 1 Gew.% Katalysator, bezogen auf Perfluoralkylethanol) vorgelegt. Zu dieser Lösung werden bei 50 °C (unter Kühlung) 309 g (3,34 mol) Epichlorhydrin zugetropft, worauf 2 h lang bei der Siedetemperatur des Lösungsmittels nachgerührt wird. Nun wird der Kolbeninhalt zur Auswaschung des Katalysators mit 1 l einer 4gew.%igen wäßrigen Natriumhydrogencarbonat-Lösung gewaschen, anschließend zweimal mit Wasser nachgewaschen und zur Entfernung des Lösungsmittels im Wasserstrahlvakuum destilliert.

Das erhaltene Perfluoralkylethanol-Epichlorhydrin-Addukt (1,77 kg; Ausbeute: 97.6 Gew.% d.Theorie) ist ein festes (wachsartiges), gelb gefärbtes Produkt (OH-Zahl 85,9); seine Bruttozusammensetzung entspricht der Formel 56.

c) Monomethylolierung

81 g (=0,05 mol) des Ausgangsproduktes der Formel 55 hergestellt nach dem vorstehenden Beispiel a), 19 g Diethylenglykoldimethylether als Lösungsmittel, 0,2 g Triethylamin als Katalysator und 3,4 g einer 45%igen (ca. 0,05 mol) Lösung von Formaldehyd in Isobutanol werden 6 h bei 55 bis 60 °C gerührt. Man erhält als Ausbeute 103 g einer bräunlichen, viskosen Lösung des Monomethylolproduktes der Formel 57, wobei ein Wasserstoff an einer -NH-Gruppe durch -$CH_2OH$ ersetzt ist, mit 82,2 % Wirksubstanz (von praktischen Molekulargewicht 1640).

Beispiel 5:

a) Herstellung einer erfindungsgemäßen kationischen Dispersion:

23,2 g Substanzgemisch nach Beispiel 4b) (mit einem Gehalt von 17,6 g an monomethyloliertem Wirkstoff, gelöst im wesentlichen in Diethylenglykoldimethylether), 1 g zusätzlichen Diethylenglykoldimethylether, 10 g Adipinsäuredibutylester, 5 g Methylpropylenglykolacetat und 2 g Tween® 80 werden bei 60 - 70 °C gelöst. Dann läßt man eine Lösung von 1,4 g der Verbindung 57

$(C_9F_{19}\text{-}CF{=}CH\text{-}CH_2\text{-}N(C_2H_5)_2CH_3)^{\oplus}SO_4CH_3^{\ominus}$ (57)

in 57,4 g Wasser unter Rühren zulaufen und behandelt 10 bis 20 min bei 70-75 °C mit Ultraschall, bis die durchschnittliche Teilchengröße unter 1 µm abgesunken ist. Man erhält 100 g einer stabilen weißlichen Emulsion mit einem Fluorgehalt von 7,2 % in der Wirksubstanz.

Diese Dispersion eignet sich vor allem zur Behandlung von Polyamidteppichen, die anschließend 3 min bei 150 °C thermosoliert werden.

Beispiel 6:

Methylolierung mit anschließender Veretherung:

81 g (= 0,05 mol) des Ausgangsproduktes der Formel 55, hergestellt nach Beispiel 4 a), 19 g Diethylenglykoldimethylether, 0,3 g Lauryldimethylamin als Katalysator und 3,8 g einer 40%igen Lösung von Formaldehyd in Isobutanol werden 2 h bei 55-60 °C gerührt. Dann setzt man 50 ml Methanol und 0,4 g Orthophosphorsäure zu und heizt weitere 4 h auf 55-60 °C. Zum Schluß wird das überschüssige Methanol im Wasserstrahlvakuum abdestilliert. Man erhält 113 g eines viskosen, etwas rotbraun verfärbten Produkts.

Beispiel 7:

Bismethylolierung eines Produktgemisches aus

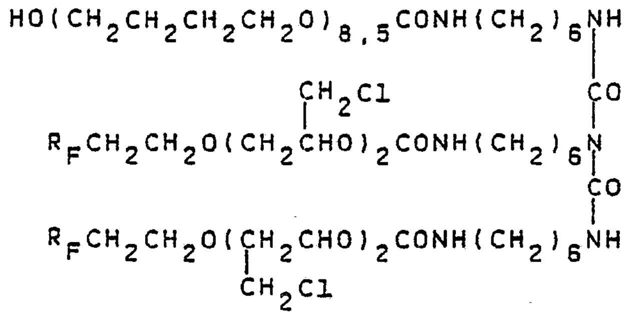

(58)

und

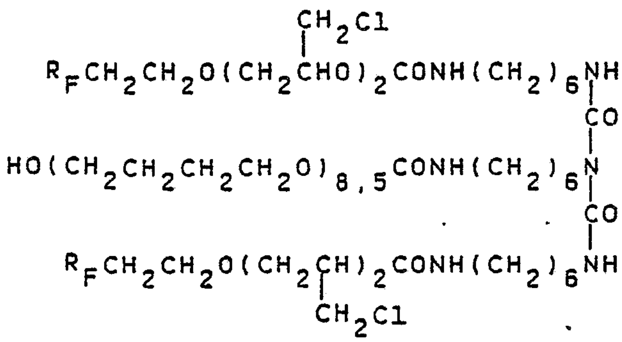

(59)

mit $R_F$ = Perfluoralkyl mit 6 bis 14 C-Atomen, erhältlich durch Umsetzung von 1 mol Desmodur N 75 mit ca. 2 mol einer Verbindung der Formel 66

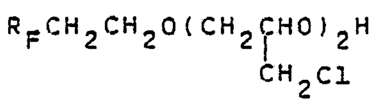

(66)

(vergl. Beispiel 13a) und 1 mol Polybutylenglykol der Formel 59a

$HO(CH_2CH_2CH_2CH_2O)_{8,5}H$ (59a).

129,6 g (= 0,05 mol) des Produktsgemisches, 32,4 g Adipinsäuredibutylester, 0,2 g DABCO und 7,4 g einer 45%igen Lösung von Formaldehyd in Isobutanol werden 4 h auf 55 bis 60 °C unter Rühren erwärmt. Man erhält 169,6 g Ausbeute an roher Bismethylolverbindung in Form einer bernsteinfarbenen, viskosen Lösung.

Beispiel 8:

Herstellung einer erfindungsgemäßen kationischen Dispersion

30 g der nach Beispiel 7 erhaltenen viskosen Lösung, 5 g Diethylenglykoldimethylether, 5 g Methoxypropylacetat und 3 g Tween® 80 werden unter Erwärmen gelöst. Dann läßt man unter Rühren eine Lösung von 3 g einer 50%igen Lösung von

$(R_F\text{-}CF=CH\text{-}CH_2\text{-}N(CH_3)_3)^{\oplus}\ Cl^{\ominus}$ (60)

mit $R_F$ Perfluoralkyl mit 6 bis 14 C-Atomen in Isopropanol, gelöst in 54 g Wasser, zulaufen und beschallt die Mischung 12 min intensiv bei 70 bis 75° C. Man erhält 100 g einer sehr schönen, weißlichen Emulsion mit einem Gehalt von 5 % an Fluor in der Wirksubstanz.

Beispiel 9:

Ausrüstung von Polyamidgewebe mit Emulsion nach Beispiel 8

2 g der Emulsion nach Beispiel 8 werden mit 98 g Wasser verdünnt. Dann tränkt man ein standardisiertes Polyamidgewebe mit dieser Lösung und quetscht auf dem Foulard ab (Abquetscheffekt 50 %). Anschließend wird 3 h bei 120° C getrocknet und schließlich 30 s bei 200° C thermosoliert.

Der Oleophobtest nach AATCC ergibt nach dem Thermosolieren eine Benotung von 6 bis 7 und zeigt damit eine um eine halbe Note verbesserte Wirksamkeit gegenüber einem nichtmethylolierten Vergleichsprodukt.

Beispiel 10:

Veretherung der Bismethylolverbindung von Beispiel 7

3,5 g Paraformaldehyd (0,11 mol) werden zunächst in einem Überschuß von 40 ml Methanol 10 min bei 50° C weitgehend vorgelöst. Dann fügt man 129,6 g (0,05 mol) des nach Beispiel 7 erhaltenen Umsetzungsprodukts, 32,4 g Adipinsäuredibutylester und 0,5 g

$$CF_3-(CF_2)_5-CH_2-CH_2-O-CO-CH_2-CH_2-N(C_4H_9)_2 \quad (61)$$

(letzteres als Katalysator) hinzu und erwärmt 2 h auf 55° C. Anschließend wird mit 0,2 ml konz. HCl sauer gestellt und weitere 3 h auf 55 bis 60° C erwärmt. Zum Schluß wird in leichtem Vakuum bei ca. 50° C das überschüssige Methanol abdestilliert. Man erhält 170 g Ausbeute an bernsteinfarbenem, viskosem Produkt (mit einem Gehalt von 5 % Fluor in der Wirksubstanz).

Beispiel 11:

Herstellung einer erfindungsgemäßen kationischen Dispersion

23,2 g des lösungsmittelhaltigen Fluorprodukts nach Beispiel 10 werden mit 7,5 g Adipinsäuredibutylester, 5 g Diethylenglykoldimethylether, 5 g Butylglykolacetat sowie 3 g eines Additionsprodukts von 100 EO (Ethylenoxideinheiten) an Nonylphenol und 0,75 g der Verbindung der Formel 62

$$(R_fC_2H_4O(CH_2\underset{CH_2Cl}{CH}O)_2CONHC_6H_{12}NHCO)_2NC_6H_{12}NHCO(OC_2H_4)_{22,9}OCH_3 \quad (62)$$

bei 100° C gelöst. Dann läßt man eine Lösung von 2,15 g einer 70%igen Lösung der Verbindung

$R_f$-$CF=CH-CH_2N^{\oplus}(CH_3)_3\ Cl^{\ominus}$ (60a)

$R_f = C_6F_{13}$-$C_{16}F_{33}$

in Isopropanol, gelöst in 54,1 g Wasser, unter gutem Rühren einlaufen und beschallt dann 12 min bei 75 bis 80 °C bis eine mittlere Teilchengröße von 1 µm oder besser etwas darunter erreicht ist. Sehr schöne, weißliche Emulsion, die monatelang stabil bleibt.

Beispiel 12:

Bismethylolierung während der gleichzeitigen Dispergierung

a) Herstellung der Ausgangsverbindung:

In einem 2-l-Schliff-Vierhalskolben mit Rührer, Tropftrichter und Kühler werden 355 g "Perfluoroctylethanol" (= 0,66 mol) mit 150 g Diethylenglykoldimethylether bei 50 °C gelöst. Dann setzt man 0,5 ml Dibutylzinndilaurat als Katalysator zu und läßt im Laufe einer halben Stunde 204 g Desmodur N 75 (= 0,33 mol) unter Rühren zutropfen. Die Temperatur im Kolben steigt durch die exotherme Reaktion bis etwa 70 °C an. Es wird noch 2 h auf 100 °C geheizt, dann läßt man die Lösung auf 60 °C abkühlen und läßt anschließend unter gutem Rühren 18 g Wasser (= 1 mol = 500 % Überschuß) eintropfen. Unter mäßiger Gas-Entwicklung entweicht das bei der Harnstoffbildung entstehende Kohlendioxid. Man läßt noch so lange (ca. 1 bis 2 Stunden) bei 100 °C nachrühren, bis praktisch alles Isocyanat verbraucht ist.
Ausbeute = 722 g einer hellen, 79%igen Lösung vom F-Gehalt = 35 %, die beim Erkalten gallertig erstarrt.

Das Produkt besitzt die Formel 63

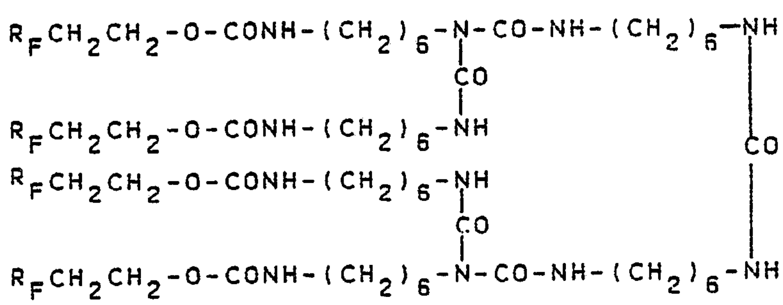

(63)

wobei $R_F$ die Bedeutung besitzt, die unter 2. vor den Beispielen angegeben ist.

b) Bismethylolierung und gleichzeitige Dispergierung:

In einem 2-l-Behälter werden 56,25 g einer 40%igen Lösung eines Emulgators der Formel 64

$(R_fCF=CHCH_2N(C_2H_5)_2CH_3)^{\oplus}\ SO_4CH_3^{\ominus}$ (64)

in Form eines technischen Gemisches mit einem F-Gehalt von 50 % in 784 g Wasser gelöst. Dann läßt man unter der starken Scherwirkung einer Dispergiermaschine vom Typ Ultraturrax im Laufe von ca. 10 min eine 80 °C warme Lösung von 300 g (= 0,07 mol) des 79%igen Produkts gemäß dem vorstehenden Beispiel a) und 45 g Tween® 80 in 300 g Butylacetat zulaufen, wobei die Temperatur im Stutzen auf ca. 45 °C ansteigt. Bei dieser Temperatur wird noch 10 min weiterdispergiert. Die hierbei entstehende Rohdispersion ist noch nicht lagerbeständig.

Man rührt nun 5 g Paraformaldehyd dazu und behandelt die Rohdispersion solange im Kreislauf mit Ultraschall oder im Hochdruckhomogenisator, bis der durchschnittliche Verteilungsgrad der Teilchen einen

mittleren Durchmesser von 1 µm unterschritten hat. Auch die Methylolierung ist dann abgeschlossen, denn freier Formaldehyd kann nicht mehr nachgewiesen werden.

Man erhält 1500 g einer stabilen, 14,3%igen, weißlichen Dispersion mit einem Fluorgehalt von 7 % und einem Feststoffgehalt von 20,2 %.

c) Bismethylolierung und gleichzeitige Dispergierung:

Bei dem vorstehend angegebenen Beispiel b) kann der eingesetzte Paraformaldehyd vorteilhafterweise durch 12 g einer 40%igen Lösung von Formaldehyd in Isobutanol ersetzt werden.

Beispiel 13:

Tetramethylolierung und Veretherung des Produktes der Formel

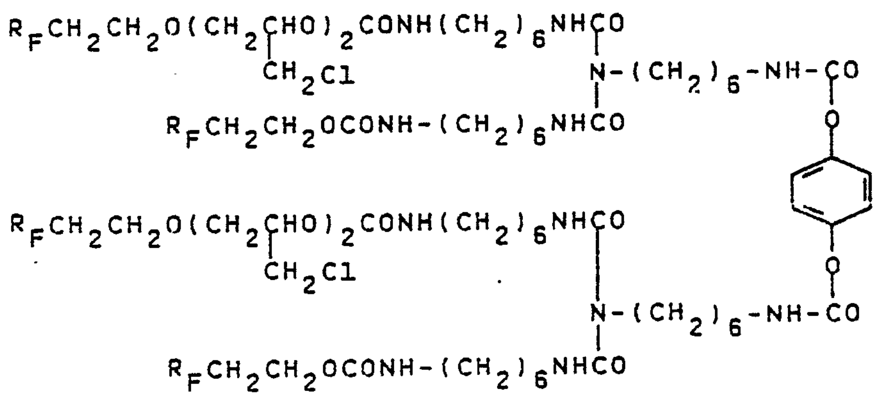

(65)

wobei $R_F$ ein Perfluoralkylgemisch von $C_8F_{17}$ bis $C_{17}F_{35}$ ist.

a) Herstellung des Ausgangsprodukts der Formel 65:

a 1) In einem mit einem Rührer, Rückflußkühler, Thermometer, Tropftrichter und Heizbad ausgestatteten Glaskolben werden 0,8 kg (1,57 mol) eines im Handel erhältlichen Perfluoralkylethanol-Gemischs mit Perfluoralkyl = $C_8F_{17}$ bis $C_{16}F_{33}$ (OH-Zahl = 106), 0,8 kg 1,2,2-Trifluortrichlorethan ($CFCl_2$-$CF_2Cl$; Kp = 48 °C) als Lösungsmittel und 5 g Bortrifluoriddiethyletherat als Katalsysator (das sind 0,6 Gew.% Katalysator, bezogen auf Perfluoralkylethanol) vorgelegt. Zu dieser Lösung werden bei 45 °C 0,29 kg (3,14 mol) Epichlorhydrin zugetropft, worauf 3 h lang bei der Siedetemperatur des Lösungsmittels weitergerührt wird. Anschließend wird das eingesetzte Lösungsmittel im Wasserstrahlvakuum abdestilliert. Es liegt ein wachsartiges gelb gefärbtes Produkt vor. In dem so erhaltenen aliphatischen Perfluoralkylethanol ist das Molverhältnis von Perfluoralkylethanol zu Epichlorhydrin = 1 : 2 (m in Formel 1 b hat den Wert 2, der sich durch Mittelwertbildung von 1 bis 8 angelagerten Epichlorhydrin-Einheiten ergibt). Das hergestellte Perfluoralkylethanol hat daher im Mittel folgende Zusammensetzung:

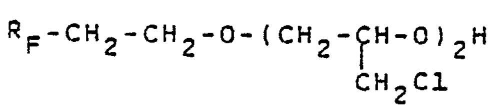

(66)

a 2) In einem mit Rührer, Rückflußkühler mit Trockenrohr, Thermometer und Heizbad ausgerüsteten Glaskolben werden vorgelegt:

162,0 g (0,32 val) des in a 1) benutzten Perfluoralkylethanol-Gemisches der Formel 67

$R_F$-$CH_2$-$CH_2OH$ (67)

216,0 g (0,32 val) des Perfluoralkylethanols der Formel 66

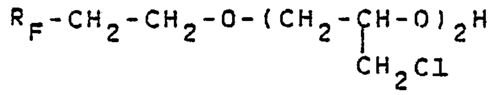 (66)

wobei $R_F$ jeweils ein Perfluoralkylgemisch von $C_8F_{17}$- bis $C_{16}F_{33}$- ist, und 181,3 g (0,96 val) des Triisocyanats der Formel 68

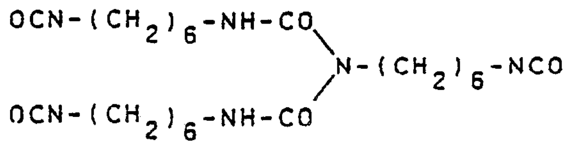 (68)

Das Triisocyanat der Formel 68 wird dabei in Form eines als Handelsprodukt erhältlichen Gemisches eingesetzt, das auch noch die Isocyanate

$OCN$-$(CH_2)_6$-$NH$-$CO$-$NH$-$(CH_2)_6$-$NCO$ (69)

$OCN$-$(CH_2)_6$-$NH$-$CO$-$NH$-$(CH_2)_6$-$NH$-$CO$-$NH$-$(CH_2)_6$-$NCO$ (70)

enthält und in dem das Triisocyanat der Formel 68 als Hauptprodukt enthalten ist.
Das Gemisch der vorgelegten Produkte (Molverhältnis 1 : 1 : 1) wird 5 h lang bei 110 °C unter Rühren gehalten. Es wird ein wachsartiges, gelb gefärbtes Produkt folgender Zusammensetzung erhalten:

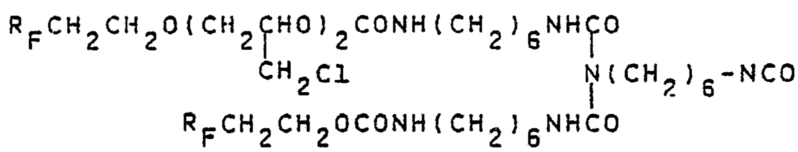 (71)

a 3) In einem mit Rührer, Rückflußkühler mit Trockenrohr, Thermometer und Heizbad ausgestatteten Glaskolben werden 559,0 g (0,32 val) der Verbindung 71, 17,5 g (0,32 val) Hydrochinon und 144,0 g Adipinsäure-di-n-butylester als Lösungsmittel vorgelegt (das ist ein Molverhältnis von 2 : 1) und 10 h lang bei 110 °C unter Rühren gehalten. Es werden 715,0 g, das sind 99,2 Gew.% der Theorie, der Verbindung der Formel 65 in Form eines wachsartigen, braun gefärbten Produktes erhalten.

b) Tetramethylolierung der Verbindung 65 und anschließende Veretherung mit Methanol:

3,5 g (= 0,117 mol) Paraformaldehyd, 40 g Methanol, 30 g Diethylenglykol-dimethylether, 73 g (0,02 mol) der Verbindung gemäß vorstehendem Beispiel a 3) und 5 g N,N-Dimethylethanolamin als Katalysator werden 3 h auf 60 bis 65 °C erwärmt. Der Paraformaldehyd geht dabei allmählich völlig in Lösung. Man setzt dann 0,5 g Orthophosphorsäure zu und erwärmt weiter 2 h auf 60 bis 65 °C. Unter leichtem Vakuum wird dann das überschüssige Methanol abdestilliert. Man erhält 110 g an rohem, bernsteinfarbenen Tetramethylolether (gelöst im Diethylenglykoldimethylether).

Beispiel 14:

Herstellung einer erfindungsgemäßen Dispersion

18,6 g des lösungsmittelhaltigen Produktes nach Beispiel 13 b) , 10 g Adipinsäuredibutylester, 5 g Methoxypropylacetat und 2,75 g des nichtionischen Coemulgators der Formel

$$(R_fC_2H_4O(CH_2\underset{\overset{|}{CH_2Cl}}{CH}O)_2CONHC_6H_{12}NHCO)_2NC_6H_{12}NHCO(OC_4H_8)_{23}OCH_3 \qquad (72)$$

werden bei 90 bis 100° C gelöst. Dann läßt man eine Lösung von 3 g einer 50 %igen Lösung von

$(R_f\text{-}CF=CH\text{-}CH_2\text{-}N(CH_3)_3)^{\oplus}\ Cl^{\ominus}$ (73)

($R_f$ bedeutet vorstehend jeweils ein Gemisch $C_6F_{13}$ bis $C_{16}F_{33}$)
in Isopropanol, gelöst in 60,65 g Wasser, unter Rühren zulaufen und beschallt die Mischung 12 min intensiv mit Ultraschall bei 70 bis 75° C. Man erhält 100 g einer sehr schönen, weißlichen Emulsion mit einem Gehalt von 5 % Fluor in der Wirksubstanz.

Beispiel 15: (Vergleichsbeispiel)

Wenn man das nach Beispiel 12 a) erhaltene Produkt ohne Methylolierung und Veretherung gemäß Beispiel 13 dispergiert, so erhält man nur eine deutlich weniger stabile Dispersion, die bereits nach 1 bis 2tägigem Stehen bei Raumtemperatur abzusetzen beginnt.

## Ansprüche

1. Methylolierte und gegebenenfalls veretherte Fluoralkylliganden enthaltende Urethane, herstellbar durch Umsetzung eines Fluoralkylliganden enthaltenden Urethans der allgemeinen Formel 1

$$(R_f\text{-}(SO_2NR^1)_a(CH_2)_x\text{-}O\text{-}(CH_2\text{-}\underset{\overset{|}{Y}}{C}H\text{-}O)_m\text{-}CO\text{-}NH)_p\text{-}R \qquad (1)$$

worin
$R_f$ eine Perfluoralkylgruppe mit 4 bis 20 C-Atomen,
$R^1$ Wasserstoff oder eine Alkylgruppe mit 1 bis 4 C-Atomen
a die Zahl 0 oder 1,
x eine Zahl von 1 bis 4,
Y Wasserstoff, $-CH_3$ oder $-CH_2Cl$,
R einen organischen Rest,
p eine Zahl von 1 bis 4,
m eine Zahl von 0 bis 4
bedeuten, mit Formaldehyd im Molverhältnis 1 : (0,5 bis 1,1 t), wobei t die Zahl der insgesamt in der Verbindung 1 vorhandenen methylolierbaren -NH-Gruppen ist, und gegebenenfalls anschließender Veretherung mit einem niederen Alkanol mit 1 bis 4 C-Atomen oder Ethylenglykolmonoalkylether mit 1 bis 4 C-Atomen im Alkylrest.

2. Urethane nach Anspruch 1, herstellbar durch Umsetzung einer Verbindung der Formel 1 mit Formaldehyd im Molverhältnis 1 : (0,5 bis (t-1)).

3. Urethane nach Anspruch 1 und/oder 2, herstellbar durch Umsetzung einer Verbindung der Formel 1 mit Formaldehyd im Molverhältnis 1 : (0,5 bis 0,525 t).

4. Urethane nach einem oder mehreren der Ansprüche 1 bis 3, dadurch gekennzeichnet, daß die Methylolierung bei Temperaturen von 20 bis 70° C, vorzugsweise 30 bis 65° C, durchgeführt wird.

5. Verfahren zur Herstellung der in einem oder mehreren der Ansprüche 1 bis 4 angegebenen methylolierten und gegebenenfalls veretherten Fluoralkylliganden enthaltenden Urethane, dadurch gekennzeichnet, daß ein Fluoralkylliganden enthaltendes Urethan der allgemeinen Formel 1 mit Formaldehyd im Molverhältnis 1 : (0,5 bis 1,1 t), wobei t die Gesamtzahl der in der Verbindung der Formel 1 vorhandenen

methylolierbaren -NH-Gruppen ist, bei Temperaturen von 20 bis 70°C umgesetzt und gegebenenfalls anschließend mit einem Alkanol mit 1 bis 4 C-Atomen oder einem Ethylenglykolmonoalkylether mit 1 bis 4 C-Atomen im Alkylrest verethert wird.

6. Verfahren nach Anspruch 5, dadurch gekennzeichnet, daß die Methylolierung im Molverhältnis Verbindung I : Formaldehyd = 1 : (0,5 bis (t-1)), vorzugsweise 1 : (0,5 bis 0,525 t), durchgeführt wird.

7. Verfahren nach Anspruch 5 und/oder 6, dadurch gekennzeichnet, daß die Methylolierung in einem organischen Lösungsmittel und/oder vorzugsweise in Anwesenheit eines basischen Katalysators durchgeführt wird.

8. Wäßrige Dispersion der methylolierten und gegebenenfalls veretherten Fluoralkylliganden enthaltenden Urethane eines oder mehrerer der Ansprüche 1 bis 4, enthaltend
5 bis 30 Gew.% Wirkstoff oder Wirkstoffgemisch, 2 bis 15 Gew.% eines oder mehrerer Dispergier- und/oder Emulgiermittel
5 bis 30 Gew.% eines Lösungsmittels oder Lösungsmittelgemisches
Rest auf 100 % Wasser.

9. Verwendung der Verbindungen nach einem oder mehreren der Ansprüche 1 bis 4 in Form ihrer Lösungen in organischen Lösungsmitteln oder in Form ihrer wäßrigen Dispersionen zur Hydrophob- und Oleophobausrüstung von Textilien oder von Leder.

Europäisches Patentamt

# EUROPÄISCHER RECHERCHENBERICHT

Nummer der Anmeldung

EP 88113293.0

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,A | US - A - 4 468 527 (KALYANJI U. PATEL)<br>* Spalten 1-4 *<br>-- | 1,8-10 | C 07 C 125/06 |
| D,A | US - A - 4 340 749 (KALYANJI U. PATEL)<br>* Patentanspruch 1; Spalte 1 *<br>-- | 1,8-10 | |
| D,A | US - A - 4 264 484 (KALYANJI U. PATEL)<br>* Spalten 1-4 *<br>---- | 1,8-10 | |

RECHERCHIERTE SACHGEBIETE (Int. Cl.4)

C 07 C

Der vorliegende Recherchenbericht wurde für alle Patentansprüche erstellt.

| Recherchenort | Abschlußdatum der Recherche | Prüfer |
|---|---|---|
| WIEN | 22-11-1988 | HEIN |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82